(19) Europäisches Patentamt
European Patent Office
Office européen des brevets

(11) **EP 3 614 904 B1**

(12) **FASCICULE DE BREVET EUROPEEN**

(45) Date de publication et mention de la délivrance du brevet:
**04.05.2022 Bulletin 2022/18**

(21) Numéro de dépôt: **18722414.2**

(22) Date de dépôt: **18.04.2018**

(51) Classification Internationale des Brevets (IPC):
**A61B 3/10** (2006.01)   **A61B 3/12** (2006.01)

(52) Classification Coopérative des Brevets (CPC):
**A61B 3/1025; A61B 3/1015; A61B 3/102; A61B 3/1225**

(86) Numéro de dépôt international:
**PCT/EP2018/059854**

(87) Numéro de publication internationale:
**WO 2018/197288 (01.11.2018 Gazette 2018/44)**

(54) **SYSTEME ET MÉTHODE D'IMAGERIE RÉTINIENNE MULTI-ECHELLE**

**SYSTEM UND VERFAHREN ZUR MULTISKALIGEN NETZHAUTBILDGEBUNG**

**SYSTEM AND METHOD FOR MULTI-SCALE RETINAL IMAGING**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **25.04.2017 FR 1753568**

(43) Date de publication de la demande:
**04.03.2020 Bulletin 2020/10**

(73) Titulaire: **Imagine Eyes**
**91400 Orsay (FR)**

(72) Inventeur: **LEVECQ, Xavier**
**91190 Gif sur Yvette (FR)**

(74) Mandataire: **Osha Liang**
**2, rue de la Paix**
**75002 Paris (FR)**

(56) Documents cités:
**WO-A1-2014/053824      WO-A1-2016/009603**
**FR-A1- 2 970 858        US-B2- 7 758 189**

## Description

## Domaine technique

La présente description concerne un système et une méthode d'imagerie rétinienne multi-échelle, plus précisément un système et une méthode d'imagerie à balayage de la rétine, permettant d'imager simultanément ou successivement des champs de la rétine de tailles différentes.

## Etat de l'art

L'imagerie à haute résolution de la rétine, c'est-à-dire à l'échelle cellulaire, permet un diagnostic précoce des maladies rétiniennes. En effet, les premiers effets des maladies rétiniennes atteignent les structures microscopiques de la rétine. Les microstructures atteintes par les trois maladies rétiniennes les plus courantes et parmi les plus graves (DMLA pour « Dégénérescence Maculaire Liée à l'Age », glaucome, rétinopathie diabétique) sont les photorécepteurs, et parmi eux les cônes qui présentent une taille variant entre 2 et 5 $\mu$m, les micro vaisseaux de la rétine qui sont les plus petit vaisseaux du corps humain (environ 6 $\mu$m de diamètre), et les cellules nerveuses qui présentent quant à elles un diamètre d'environ 2 $\mu$m.

Plusieurs laboratoires proposent différents systèmes d'imagerie permettant de faire de l'imagerie rétinienne avec une résolution cellulaire. Ces différents systèmes, également appelés « ophtalmoscopes à optique adaptative » mettent en œuvre des modules d'éclairage et/ou détection de la rétine différents mais tous comprennent un module d'optique adaptative permettant de mesurer les défauts optiques de l'œil et du système d'imagerie et de corriger les rayons lumineux issus de la rétine et incidents sur le système de détection pour gagner en résolution.

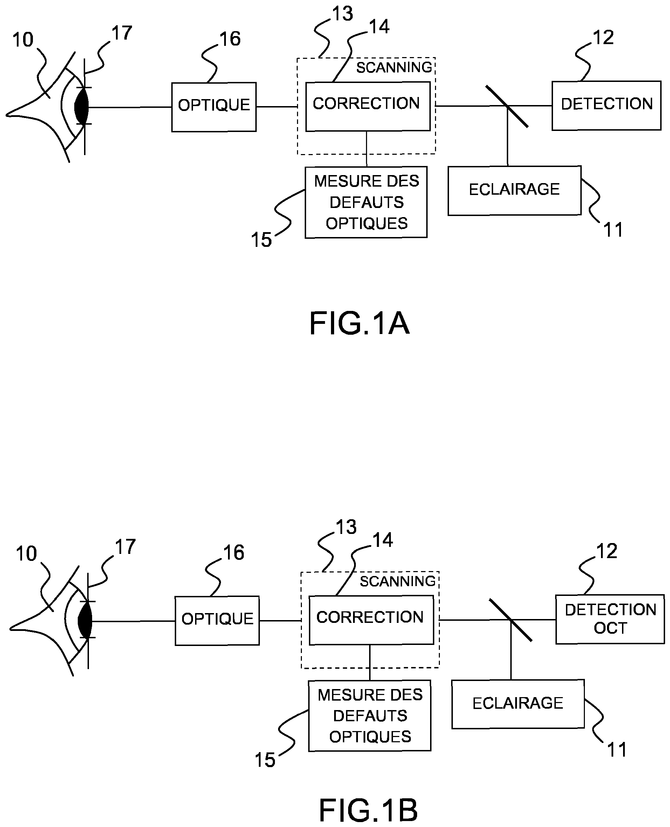

La figure 1A représente un schéma de principe d'un système d'imagerie rétinienne basée sur la technologie d'ophtalmoscopie à balayage laser avec optique adaptative ou 'AOSLO' (abréviation de l'expression anglo-saxonne 'Adaptive Optics Scanning Laser Ophtalmoscopy'), tel que décrit par exemple dans l'article de A. Roorda et al. (« Adaptive optics scanning laser ophtalmoscopy », Optics express 405, Vol. 10, N°9, 2002). Le montage de l'AOSLO comprend principalement un module d'éclairage 11 de la rétine, un module de détection 12, un module de balayage (« scanning ») 13, un module de correction 14 comprenant un plan de correction des rayons lumineux incidents, un module d'analyse du front d'onde 15 et un système optique d'imagerie 16. Le module d'éclairage comprend par exemple une diode laser couplée à une fibre optique pour former un point source et une lentille optique permettant de former à partir du point source un faisceau d'éclairage. Un diaphragme du module d'éclairage 11 définit une pupille. Le faisceau d'éclairage est envoyé, par exemple par un jeu de miroirs (non représentés), sur le module de correction 14, par exemple un miroir déformable, puis dans le module de balayage 13 pour être dirigé selon un balayage vertical et horizontal dans l'œil 10 d'un sujet. Le faisceau d'éclairage est ainsi focalisé pour former sur la rétine un faisceau quasi ponctuel qui balaye la rétine et la lumière, par exemple rétrodiffusée par la rétine, subit le même balayage optique au retour pour être envoyée sur le miroir déformable 14 et le module de détection 12, comprenant par exemple un trou de détection confocale et un détecteur qui peut être un photomultiplicateur ou une photodiode à avalanche. Un ensemble d'éléments optiques symbolisé par le système d'imagerie 16 participe à conjuguer optiquement le plan de la rétine et le trou de détection confocale du détecteur. Le module d'analyse du front d'onde 15 comprend par exemple un analyseur de type Shack-Hartmann ; il reçoit la lumière rétrodiffusée par la rétine et contrôle le miroir déformable afin de corriger le faisceau d'éclairage et le faisceau rétrodiffusé. Le plan de la pupille du module d'éclairage, le plan du miroir déformable et un plan d'analyse du module d'analyse du front d'onde sont conjugués optiquement avec un plan prédéterminé 17 de l'œil, par exemple le plan pupillaire de l'œil.

La figure 1B représente un schéma de principe d'un montage de type OCT (abréviation de l'expression anglo-saxonne 'Optical Coherence Tomography') couplé à de l'optique adaptative. Un tel système est décrit par exemple dans R. Zawadzki (« Adaptive-optics optical coherence tomography for high resolution and high speed 3D retinal in vivo imaging » Optics Express 8532, Vol. 13, N°21, 2005). L'OCT repose sur l'utilisation d'un interféromètre à faible cohérence. Cette technique d'imagerie permet de réaliser in vivo des images en coupe de tissus, avec une résolution de quelques microns. Un intérêt de l'OCT en ophtalmologie provient de sa capacité à révéler in-vivo des tissus au travers d'autres tissus diffusants. Le montage de la figure 1B reprend de façon très simplifiée les éléments principaux d'un montage de type OCT. On retrouve un arrangement similaire à celui de l'AOSLO mais dans lequel le module de détection 12 est spécifique à l'OCT et comprend notamment un interféromètre, par exemple un interféromètre fibré, par exemple de type Michelson. Le point d'entrée de la fibre (non représentée) est conjugué avec la rétine de l'œil 10 au moyen d'un système optique de conjugaison symbolisé par l'optique 16. En comparaison avec l'AOSLO, la technologie OCT permet d'avoir une coupe longitudinale de la rétine au détriment de la vitesse d'acquisition.

Cependant, les ophtalmoscopes à optique adaptative décrits ci-dessus, s'ils présentent une excellente résolution, sont limités à l'imagerie d'un champ limité de la rétine, typiquement un champ de quelques degré (inférieur à 4°x4° typiquement) ; ceci est dû au fait que le champ d'isoplanétisme de l'œil ne permet pas la correction des aberrations sur un champ beaucoup plus grand avec un simple système d'optique adaptative.

L'article d'A. Dubra et al. (« First-order design of a reflective viewfinder for adaptive optics ophtalmoscopy », Optics Express, Vol. 20, N°24 (2012)) décrit un ophtalmoscope à optique adaptative de type AOSLO avec un agencement optique particulier, dans lequel un système optique relai amovible permet de basculer entre un mode « petit-champ » présentant une grande résolution et un mode « grand-champ », de plus faible résolution. Ainsi, la mise en place du système optique relai amovible permet de basculer vers un arrangement optique « grand-champ » présentant un grandissement angulaire plus grand au détriment d'un grandissement pupillaire plus faible. Le mode grand-champ permet à un utilisateur de rechercher les zones d'intérêt de la rétine. Le système optique relai amovible peut alors être retiré pour revenir au mode petit-champ adapté à l'imagerie rétinienne de grande résolution.

En pratique, un tel système est cependant difficilement envisageable pour un produit commercial. En effet, la mise en place d'un système optique relai comprenant un ensemble de plusieurs éléments de conjugaison optique nécessite de réajuster l'ensemble des réglages optiques, ce qui est une opération délicate et souvent longue, peu adapté à un utilisateur non expérimenté.

La demande de brevet WO 2016/009603 décrit également un système d'imagerie rétinienne de type AOSLO adapté à une imagerie petit-champ et grand-champ. Plus précisément, le système décrit comprend deux unités adaptées respectivement à l'imagerie grand-champ basse résolution et petit-champ grande résolution, les deux unités étant séparés par une lame séparatrice. Comme chaque unité comprend un module d'éclairage et de détection, associé à un module de balayage spécifique, ainsi qu'un système d'imagerie propre, un utilisateur peut avoir accès à la fois à l'imagerie petit-champ et grand-champ sans aucun réglage et de façon simultanée. Ce système suppose cependant de doubler les modules d'éclairage et de détection ainsi que les modules de balayage pour la formation des unités grand-champ et petit-champ, ce qui n'est pas satisfaisant.

Le brevet US 7,758,189 décrit un système pour l'imagerie de la rétine avec une première unité grand-champ de type LSLO (pour « *Line-scanning laser ophtalmoscope* ») et une deuxième unité petit-champ de type AO-SDOCT (pour « *Adaptive Optics Spectral Domain Optical Coherence Tomography* »). La demande de brevet WO2014/053824 décrit un système pour l'imagerie de la rétine avec une première unité grand champ UWF-SLO (pour «*ultra wide field* SLO) et une unité OCT distincte de la voie UWF SLO. Dans ces documents, comme dans la demande WO2016/009603 mentionnée ci-dessus, chaque unité comprend un module d'éclairage et de détection associé à un module de balayage spécifique, ainsi qu'un système d'imagerie propre.

Un objet de la présente description est de proposer un système d'imagerie à balayage de la rétine multi-échelle, c'est-à-dire adapté à une imagerie grand-champ et petit-champ qui ne présente pas les limitations de l'art antérieur. Plus précisément, un objet de la présente description est de proposer un système d'imagerie à balayage de la rétine multi-échelle dans lequel l'imagerie petit-champ et l'imagerie grand-champ peuvent être réalisées avec le même module d'éclairage et de détection et le même module de balayage, mais qui puisse être utilisé sans difficulté par un utilisateur peu expérimenté.

RESUME DE L'INVENTION

Selon un premier aspect, la présente description concerne un système d'imagerie à balayage de la rétine multi-échelle comprenant :

- au moins un premier module d'éclairage et de détection configuré pour l'émission d'au moins un premier faisceau d'éclairage avec un diamètre de dimension donnée et la détection d'au moins un premier faisceau réémis par la rétine;
- au moins un premier module de balayage du au moins un premier faisceau d'éclairage et du au moins un premier faisceau réémis;
- une première voie optique, dite voie « grand champ », pour la focalisation du au moins un premier faisceau d'éclairage sur la rétine et la réception du au moins un premier faisceau réémis par la rétine, comprenant :
  o un premier système optique avec un premier grandissement, configuré pour conjuguer un plan situé à proximité d'un plan de rotation du au moins un premier module de balayage et le plan de la pupille d'entrée de l'œil;
- une deuxième voie optique, dite voie « petit champ », pour la focalisation du au moins un premier faisceau d'éclairage sur la rétine et la réception du au moins un premier faisceau réémis par la rétine, comprenant :

  o un dispositif de correction de front d'onde présentant une surface utile de dimension donnée;
  ∘ un deuxième système optique avec un deuxième grandissement configuré pour conjuguer un plan situé à proximité d'un plan de rotation du au moins un premier module de balayage et la surface utile du dispositif de correction de front d'onde,
  ∘ un troisième système optique avec un troisième grandissement, comprenant au moins une partie du premier système optique, configuré pour conjuguer ladite surface utile du dispositif de correction et le plan de la pupille d'entrée de l'œil ;

- au moins un premier élément optique de renvoi, au moins partiellement réfléchissant, configuré pour envoyer le au moins un premier faisceau réémis par la rétine sur l'une et/ou l'autre des première et deuxième voies d'imagerie, et destiné à être positionné sur la première voie d'imagerie, entre la partie commune des premier et troisième systèmes optiques et le au moins un premier module de balayage, et sur la deuxième voie d'imagerie, entre la partie commune des premier et troisième systèmes optiques et le dispositif de correction de front d'onde.

La déposante a montré qu'un système d'imagerie ainsi décrit permet une imagerie petit-champ et/ou grand-champ avec un seul module d'éclairage et de détection et un seul module de balayage pour les deux voies, et une très grande simplicité d'utilisation. En effet, l'agencement particulier dudit premier élément optique de renvoi dans le système permet de définir le grandissement pupillaire spécifique à chaque voie par des éléments fixes du système. Aucun réglage sur des éléments optiques avec puissance optique n'est donc nécessaire lors du passage d'une voie d'imagerie à l'autre.

Ledit au moins un premier élément optique de renvoi peut être une simple surface réfléchissante amovible, par exemple un miroir amovible, configuré pour basculer entre l'une et l'autre des première et deuxième voies d'imagerie.

Ledit au moins un premier élément optique de renvoi peut également être une lame dichroïque, configuré pour envoyer le au moins un premier faisceau réémis par la rétine sur l'une desdites première et deuxième voies d'imagerie et envoyer au moins un deuxième faisceau réémis par la rétine, de longueur d'onde différente de celle dudit au moins un premier faisceau réémis par la rétine, sur l'autre voie d'imagerie. Cette configuration nécessite de travailler avec plusieurs faisceaux de longueurs d'onde différentes (et donc deux sources et potentiellement deux détecteurs dans le module d'éclairage et de détection) mais présente l'avantage de permettre une imagerie simultanée petit-champ et grand-champ avec un seul module de balayage. Les images dans les deux champs, petit-champ et grand-champ, peuvent donc être réalisées en simultanée, par le même module de balayage ; elles ont donc la même référence angulaire, ce qui rendant immédiat la colocalisation des deux images.

Selon un ou plusieurs exemples de réalisation, le premier élément optique de renvoi, par exemple une surface réfléchissante amovible ou une lame dichroïque, est configuré en outre pour envoyer le au moins un premier faisceau d'éclairage sur l'une et/ou l'autre des première et deuxième voies d'imagerie. Dans ce cas, seul un élément optique de renvoi est nécessaire dans le système d'imagerie, ce qui le rend particulièrement simple de mise en œuvre.

Selon un ou plusieurs exemples de réalisation, un deuxième élément optique de renvoi, par exemple une deuxième surface réfléchissante amovible ou une lame dichroïque, est configuré pour envoyer le au moins un premier faisceau d'éclairage sur l'une et/ou l'autre des première et deuxième voies d'imagerie.

Selon un ou plusieurs exemples de réalisation, ledit premier module d'éclairage et de détection est configuré pour une imagerie de la rétine de type AOSLO. Ledit premier module d'éclairage et de détection comprend dans cet exemple au moins une source lumineuse ponctuelle et au moins un système de détection confocale.

Selon un ou plusieurs exemples de réalisation, ledit premier module d'éclairage et de détection est configuré pour une imagerie de la rétine de type OCT. Ledit premier module d'éclairage et de détection comprend au moins une source lumineuse ponctuelle et au moins un interféromètre pour la détection.

Selon un ou plusieurs exemples de réalisation, le système d'imagerie à balayage de la rétine multi-échelle comprend un premier module d'éclairage et de détection et un deuxième module d'éclairage et de détection, chacun associé à un module de balayage. Par exemple, le premier module d'éclairage et de détection est configuré pour une imagerie de la rétine de type AOSLO et le deuxième module d'éclairage et de détection est configuré pour une imagerie de la rétine de type OCT, permettant de bénéficier de deux types d'imagerie, avec le même système, soit en mode petit-champ, soit en mode grand-champ.

Selon un ou plusieurs exemples de réalisation, le module de balayage est un module de balayage bidimensionnel, permettant un balayage dans deux directions différentes. Le module de balayage peut également être un module de balayage à une dimension, par exemple dans le cas de l'émission d'une ligne de lumière réalisée à l'aide d'une lentille cylindrique par exemple et de la réception par une barrette de détecteurs.

Ainsi, selon un ou plusieurs exemples de réalisation, le système d'imagerie à balayage de la rétine multi-échelle comprend en outre :

- un deuxième module d'éclairage et de détection configuré pour l'émission d'au moins un deuxième faisceau d'éclairage avec un diamètre de dimension donnée et la détection d'au moins un deuxième faisceau réémis par la rétine;
- un deuxième module de balayage du au moins un deuxième faisceau d'éclairage et du au moins un deuxième faisceau réémis.

Selon un ou plusieurs exemples de réalisation, le système d'imagerie à balayage de la rétine multi-échelle comprend en outre un module d'analyse du front d'onde configuré pour l'analyse d'au moins une partie des défauts optiques d'un faisceau réémis par la rétine et envoyé sur la deuxième voie d'imagerie.

Dans la suite de la description, on entend par « défauts optiques » les perturbations que subissent les rayons lumineux entre la rétine et un détecteur d'un module d'éclairage ou de détection. Ces défauts comprennent par exemple les défauts apportés par le système optique de l'œil mais aussi par au moins une partie des optiques de la voie d'imagerie petit-champ.

Selon un ou plusieurs exemples de réalisation, le module d'analyse du front d'onde comprend un analyseur de type Shack-Hartmann. Un tel dispositif permet d'analyser, par rapport à des directions nominales, la variation des directions des rayons lumineux après avoir traversé le système optique entaché de défauts optiques. Un tel dispositif réalise cette mesure grâce, par exemple, à l'agencement d'un détecteur matriciel dans le plan focal d'une matrice de microlentilles. Les variations ainsi mesurées peuvent être directement exploités pour le contrôle du dispositif de correction du front d'onde.

Selon un ou plusieurs exemples de réalisation, le système d'imagerie à balayage multi-échelle comprend en outre un module d'éclairage configuré pour l'émission d'un faisceau d'éclairage de la rétine en vue de la mesure du front d'onde.

Selon un ou plusieurs exemples de réalisation, le dispositif de correction du front d'onde comprend un miroir déformable, un modulateur spatial de lumière à cristaux liquides (SLM pour « spatial light modulator »), un MEMs (systèmes micro électromécaniques), une lentille liquide multi actionneurs. Le dispositif de correction de front d'onde peut être contrôlé par un module d'analyse du front d'onde, par exemple dans une configuration d'asservissement en boucle fermée, ou être contrôlé à partir d'algorithmes basés sur un critère de qualité des images rétiniennes acquises par le module d'éclairage et de détection.

Selon un deuxième aspect, la présente description concerne une méthode d'imagerie par balayage de la rétine au moyen d'un système d'imagerie selon le premier aspect.

Selon un ou plusieurs exemples de réalisation, ledit au moins un premier élément optique de renvoi est une surface réfléchissante amovible et la méthode comprend le retrait dudit au moins un premier élément optique de renvoi pour basculer de l'une desdites première et deuxième voies d'imagerie à l'autre voie d'imagerie.

Selon un ou plusieurs exemples de réalisation, ladite surface réfléchissante est insérée ou retirée selon un mouvement d'insertion ou de retrait parallèle à la surface réfléchissante ; cela permet d'éviter qu'une erreur de positionnement lors de l'arrêt du miroir puisse avoir une incidence sur l'alignement d'un faisceau réfléchi par ladite surface réfléchissante.

Selon un ou plusieurs exemples de réalisation, ledit au moins un premier élément optique de renvoi est une lame dichroïque et la méthode comprend l'envoi du au moins un premier faisceau réémis par la rétine sur l'une desdites première et deuxième voies d'imagerie et l'envoi d'au moins un deuxième faisceau réémis par la rétine sur l'autre voie d'imagerie, lesdits premier et deuxième faisceaux réémis par la rétine ayant des longueurs d'onde différentes.

## BREVE DESCRIPTION DES DESSINS

D'autres avantages et caractéristiques de l'invention apparaîtront à la lecture de la description, illustrée par les figures suivantes :

- FIGS. 1A et 1B (déjà décrites), des schémas de principe de systèmes d'imagerie à balayage de la rétine connus de l'art antérieur ;

- FIGS. 2A - 2C, des schémas illustrant le fonctionnement d'un premier exemple de réalisation d'un système d'imagerie à balayage de la rétine selon la présente description;

- FIGS. 3A, 3B, des schémas montrant des exemples de modules d'éclairage et de détection, adaptés à une imagerie de la rétine respectivement de type AOSLO et de type OCT ;

- FIG. 4, un schéma le fonctionnement d'un autre exemple de réalisation d'un système d'imagerie à balayage de la rétine selon la présente description ;

- FIGS 5A - 5B, des schémas illustrant le fonctionnement d'un autre exemple de réalisation d'un système d'imagerie à balayage de la rétine selon la présente description.

Par souci de cohérence, les éléments identiques sont repérés par les mêmes références dans les différentes figures.

## DESCRIPTION DETAILLEE

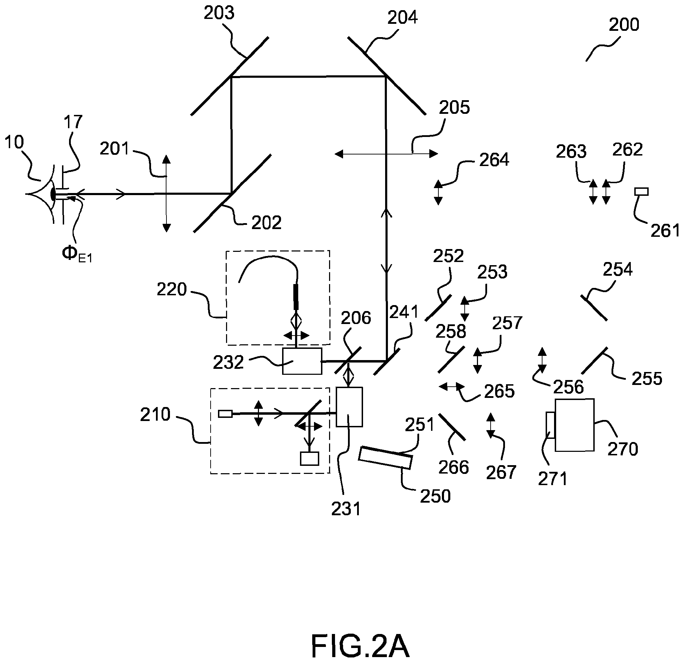

Les FIGS 2A à 2C illustrent le fonctionnement d'un premier exemple de réalisation d'un système d'imagerie à balayage de la rétine multi-échelle.

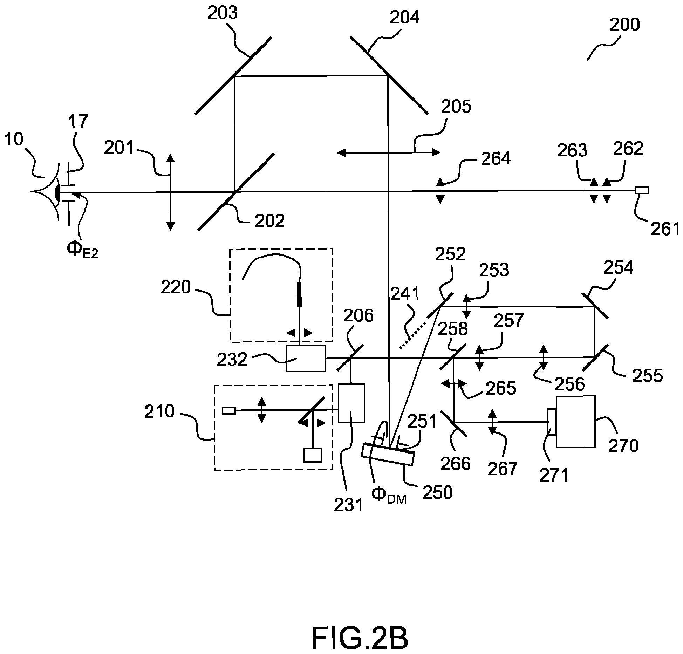

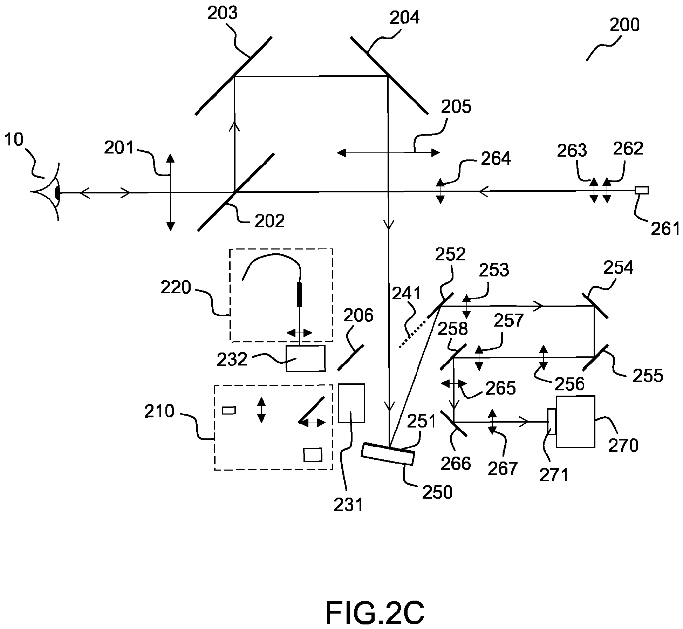

Dans cet exemple, un simple élément optique de renvoi amovible 241, par exemple un miroir plan amovible réfléchissant aux longueurs d'onde des différents faisceaux d'éclairage et faisceaux réémis par la rétine, permet de basculer entre une voie d'imagerie grand-champ (FIG. 2A) et une voie d'imagerie petit-champ (FIG. 2B). La FIG. 2C illustre l'analyse des défauts optiques dans le cas de l'imagerie petit-champ.

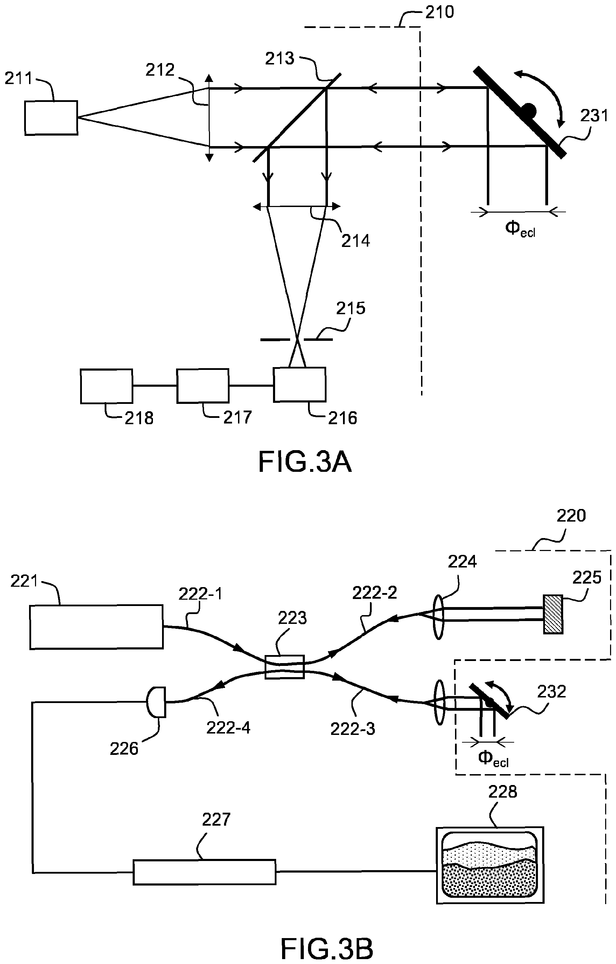

Le système d'imagerie 200 représenté sur les FIGS 2A - 2C comprend un premier module d'éclairage et de détection 210 et un deuxième module d'éclairage et de détection 220. Des exemples de tels modules d'éclairage et de détection seront décrits plus en détails en référence aux FIGS 3A -3B. Chaque module d'éclairage et de détection est adapté pour l'émission d'un ou plusieurs faisceaux d'éclairage et la détection d'un ou plusieurs faisceaux réémis par la rétine après illumination par lesdits faisceaux d'éclairage. Un faisceau réémis par la rétine peut être un faisceau rétrodiffusé à la même longueur d'onde que celle du faisceau d'éclairage, ou réémis à une longueur d'onde différente lorsque le faisceau d'éclairage induit par exemple un mécanisme de fluorescence au niveau de la rétine.

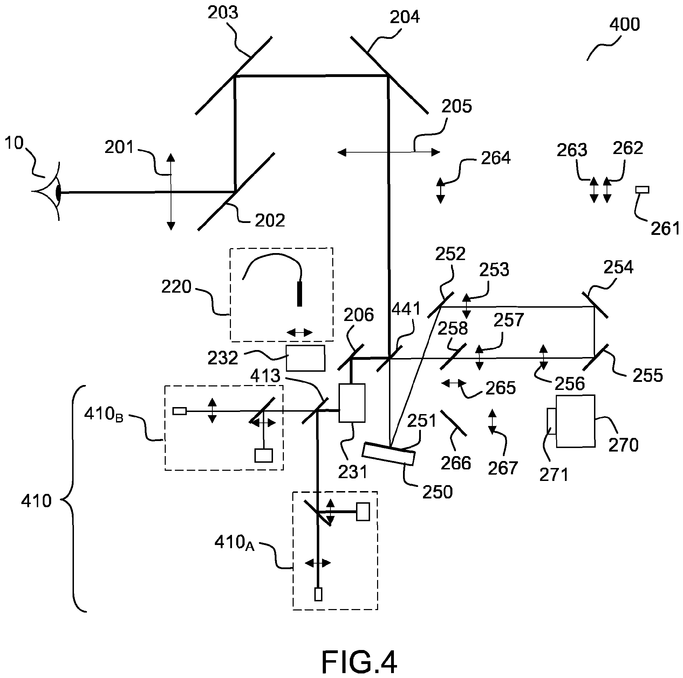

Par exemple, un module d'éclairage et de détection peut être adapté pour l'émission de deux faisceaux d'éclairage de la rétine, à des longueurs d'onde différentes, comme cela sera décrit par la suite en référence à la FIG. 4. Alternativement, comme dans le cas par exemple des FIGS. 2A - 2C, les modules d'éclairage et de détection sont adaptés pour l'émission d'un faisceau d'éclairage à une longueur donnée et la détection d'un faisceau réémis par la rétine, à cette même longueur d'onde, ou à une longueur d'onde différente dans le cas de réémission de fluorescence par la rétine.

Le système d'imagerie 200 comprend en outre, associé à chacun des modules d'éclairage et de détection 210, 220, un module de balayage, par exemple adapté à un balayage bidimensionnel, référencé respectivement 231, 232.

Chaque module de balayage est adapté pour le balayage du faisceau d'éclairage émis par le module d'éclairage et de détection et le balayage du faisceau réémis par la rétine pour être envoyé vers le module d'éclairage et de détection. Un module de balayage ou « scanner » comprend par exemple une combinaison de miroirs motorisés au moyens de moteurs galvanométriques ou une combinaison de miroir MEMS (à un axe) ou un miroir MEMS à deux axes. Pour chaque module de balayage, on peut définir un plan de rotation qui comprend l'axe de rotation ; un plan de rotation est alors confondu avec le plan du miroir. Dans le cas où on utilise deux miroirs distincts pour réaliser les deux rotations, il est avantageux que les plans de rotation de ces deux miroirs soient conjugués optiquement, par exemple au moyen d'un système optique qui conjugue les deux plans. Dans le cas où le module de balayage est un module de balayage bidimensionnel comprenant deux miroirs de balayage distincts et non conjugués, il est avantageux qu'ils soient situés à la distance la plus courte possible l'un de l'autre.

Dans l'exemple des FGS 2A - 2C, les faisceaux d'éclairage émis respectivement par les modules d'éclairage et de détection 210, 220 et balayés par les modules de balayage 231 et 232 respectivement sont combinés et envoyés vers les voies d'imagerie au moyen d'une lame partiellement réfléchissante 206, par exemple une lame dichroïque si les faisceaux d'éclairage ont des longueurs d'onde différentes. La même lame partiellement réfléchissante permet de séparer les faisceaux réémis par la rétine vers chacun des modules d'éclairage et de détection.

Le système d'imagerie 200 comprend selon la présente description une première voie optique, dite voie « grand-champ », et une deuxième voie optique, dite voie « petit-champ », l'une ou l'autre de ces voies pouvant être activée, dans l'exemple des FIGS. 2A - 2C, grâce à la mise en place ou le retrait de la surface réfléchissante amovible 241 ; et chacune desdites voies permettant la focalisation sur la rétine du faisceau d'éclairage issu du module d'éclairage et de détection et la réception du faisceau réémis par la rétine.

La FIG. 2A représente le cas où la voie grand-champ est activée. Pour ce faire, dans cet exemple, la surface réfléchissante amovible 241 est mise en place. Comme il peut s'agir d'un simple miroir, par exemple un miroir plan, aucun réglage n'est nécessaire. Par exemple, le miroir est inséré selon un mouvement d'insertion parallèle à la surface réfléchissante, évitant ainsi qu'une erreur de positionnement lors de l'arrêt du miroir puisse avoir une incidence sur l'alignement d'un faisceau réfléchi par ladite surface réfléchissante.

La voie grand-champ comprend un premier système optique, comprenant dans cet exemple un ensemble d'éléments optiques 201, 205, et présentant un premier grandissement $g_1$. Le premier système optique est destiné à conjuguer un plan positionné à proximité d'un plan de rotation du module de balayage et le plan 17 de la pupille d'entrée de l'œil 10 d'un patient.

Dans le cas où le module de balayage comprend un miroir avec un plan de rotation, ou deux miroirs conjugués optiquement l'un avec l'autre, le premier système optique pourra conjuguer le ou un desdits plans de rotation avec le plan 17 de la pupille d'entrée de l'œil 10. Dans le cas où le module de balayage est un module de balayage bidimensionnel comprenant deux miroirs de balayage distincts et non conjugués, le premier système optique pourra conjuguer un plan situé entre les deux miroirs, par exemple un plan situé à une distance médiane des deux miroirs, avec le plan 17 de la pupille d'entrée de l'œil 10.

La voie grand-champ comprend, par ailleurs, un ensemble d'éléments optiques de renvoi, dans cet exemple 204, 203, 202, pour diriger le faisceau d'éclairage vers l'œil 10.

Ainsi, dans le cas de l'imagerie « grand-champ », le diamètre $\Phi_{E1}$ de la pupille de sortie du système optique d'imagerie au niveau du plan 17 de la pupille de l'œil est donné par :

$$\Phi_{E1} = g_1 \, \Phi_{ecl} \qquad (1)$$

Le « système optique d'imagerie » est défini par l'ensemble des éléments d'imagerie entre la pupille d'entrée de l'œil et un détecteur d'un module d'éclairage et de détection et $\Phi_{ecl}$ est le diamètre du faisceau d'éclairage en sortie du module de balayage.

A noter que dans le cas où il y a deux modules d'éclairage et de détection, il peut y avoir des faisceaux d'éclairage de diamètres différents et donc des valeurs $\Phi_{E1}$ différentes, bien qu'on cherchera à rendre cette différence minimale.

On entend par « pupille » d'un système optique, la plus petite ouverture qui limite l'entrée ou la propagation des rayons lumineux dans le système. Cette ouverture peut être réelle dans le cas où un diaphragme physique, pupille du système optique considéré, limite l'entrée des rayons lumineux ou virtuelle dans le cas où cette ouverture est une image de la pupille physique du système optique qui se trouve à l'intérieur du système optique et qui est formé par exemple par un diaphragme. Ainsi, dans le cas où la pupille de sortie du système optique d'imagerie est positionnée dans le plan pupillaire de l'œil ou dans un plan se situant à proximité de ce dernier, ladite pupille de sortie est virtuelle, image d'un diaphragme physique situé à l'intérieur dudit système optique d'imagerie.

La FIG. 2B représente le cas où la voie petit-champ est activée. Pour ce faire, dans cet exemple, la surface réfléchissante amovible 241 est retirée.

La voie « petit champ » comprend un dispositif de correction de front d'onde 250 présentant une surface utile 251 de dimension donnée, un deuxième système optique, destiné à conjuguer un plan situé à proximité d'un plan de rotation du module de balayage et la surface utile 251 et présentant un deuxième grandissement $g_2$. Le deuxième système optique comprend dans l'exemple des FIGS. 2A - 2C un ensemble d'éléments optiques 257, 256, 253.

Comme pour la voie grand-champ, dans le cas où le module de balayage comprend un miroir avec un plan de rotation, ou deux miroirs conjugués optiquement l'un avec l'autre, le deuxième système optique pourra conjuguer le ou un desdits plans de rotation avec la surface utile 251 du dispositif de correction de front d'onde. Dans le cas où le module de balayage est un module de balayage bidimensionnel comprenant deux miroirs de balayage distincts et non conjugués, le deuxième système optique pourra conjuguer un plan situé entre les deux miroirs, par exemple un plan situé à une distance médiane des deux miroirs, avec la surface utile 251 du dispositif de correction de front.

La voie « petit champ » comprend en outre un troisième système optique adapté pour conjuguer la surface utile 251 du dispositif de correction et le plan 17 de la pupille d'entrée de l'œil 10, présentant un troisième grandissement $g_3$. Le troisième système optique comprend au moins une partie du premier système optique; dans l'exemple des FIGS 2A - 2C, le troisième système optique comprend tous les éléments optiques du premier système optique, à savoir les éléments optiques 205, 201 ; le troisième système optique est donc confondu avec le premier système optique ($g_3 = g_1$).

La voie petit-champ comprend par ailleurs, un ensemble d'éléments optiques de renvoi, dans cet exemple 255, 254, 252, pour diriger le faisceau d'éclairage vers l'œil 10 d'un patient.

Ainsi, dans le cas de l'imagerie « petit-champ », le diamètre $\Phi_{E2}$ de la pupille de sortie du système optique d'imagerie au niveau du plan 17 de la pupille de l'œil, est donné par :

$$\Phi_{E2} = g_3\ \Phi_{DM} \qquad (2)$$

Où $\Phi_{DM}$ est le plus petit des deux diamètres entre le diamètre de la surface éclairée du dispositif de correction de front d'onde et le diamètre de la surface utile du dispositif de correction de front d'onde, et vérifie:

$$\Phi_{DM} = g_2\ \Phi_{ecl} \qquad (3)$$

En général, pour un système dimensionné pour optimiser la résolution, $\Phi_{DM}$ sera le diamètre de la surface utile du dispositif de correction de front d'onde.

Ainsi, il en résulte le grandissement suivant entre le diamètre du faisceau d'éclairage en sortie du module de balayage et le diamètre $\Phi_{E2}$ de la pupille de sortie du système optique d'imagerie au niveau du plan 17 de la pupille de l'œil :

$$\Phi_{E2} = g_3\cdot g_2\ \Phi_{ecl} \qquad (4)$$

A partir des équations (1) et (4), on constate qu'il est ainsi possible de changer la dimension du diamètre de la pupille de sortie du système optique d'imagerie au niveau du plan 17 de la pupille de l'œil par une simple mise en place ou retrait de la surface réfléchissante 241.

Avec une plus grande pupille (FIG. 2B), pour un même angle de balayage au niveau du ou des miroirs de balayage, le champ est plus petit mais la résolution plus grande. En effet, la limite de résolution est directement proportionnelle à la taille de la pupille. D'autre part, l'angle de balayage au niveau de la pupille d'entrée dans l'œil est égal à l'angle de balayage au niveau du ou des miroirs de balayage multiplié par le grossissement du système optique entre le ou les miroirs de scan et la pupille d'entrée dans l'œil. Or le grossissement est l'inverse du grandissement. Par conséquent si la pupille d'entrée est plus grande d'un facteur X dans le cas de l'imagerie petit-champ par rapport à la pupille d'entrée dans l'œil du cas de l'imagerie grand-champ, l'angle de balayage au niveau de la pupille d'entrée dans l'œil pour l'imagerie petit-champ est plus petit du même facteur X que l'angle de balayage au niveau de la pupille d'entrée dans l'œil pour l'imagerie grand-champ (pour le même angle de balayage physique du ou des miroirs de balayage). Une réduction de l'angle de balayage au niveau de la pupille d'entrée dans l'œil pour l'imagerie petit-champ est tout à fait acceptable car lorsqu'on cherche de la haute résolution (cas de l'imagerie petit-champ), le champ d'isoplanétisme de l'œil réduit de toute façon le champ de l'image à quelques degrés (typiquement 4°x4°).

Dans l'exemple des FIGS 2A - 2C, le système d'imagerie 200 comprend en outre un module d'analyse du front d'onde adapté pour l'analyse des défauts optiques d'un faisceau réémis par la rétine et envoyé sur la deuxième voie d'imagerie. La FIG. 2C illustre plus particulièrement le chemin des faisceaux optiques pour l'analyse des défauts optiques (voie d'analyse).

Il s'agit d'analyser des perturbations que subissent les rayons lumineux entre la rétine et un détecteur du module d'éclairage et de détection, et plus précisément, entre la rétine et un plan d'analyse de l'analyseur de front d'onde. Les défauts optiques au sens de cette description comprennent donc les défauts apportés par le système optique de l'œil mais aussi par la partie du système optique d'imagerie commune avec la voie d'analyse. A noter cependant que les éventuels défauts de la voie d'analyse peuvent avoir été mesurés lors de la fabrication et la correction peut en tenir compte. Le module d'analyse du front d'onde comprend par exemple un analyseur 270 de type Shack-Hartmann (HASO® 32-eye Imagine Eyes®), comprenant un plan d'analyse 271 formé d'un ensemble de microlentilles et un détecteur disposé dans le plan focal des microlentilles.

Dans l'exemple des FIGS 2A - 2C, une lame partiellement réfléchissante 258 permet de prélever un faisceau réémis par la rétine pour l'envoyer vers le module d'analyse du front d'onde. Le module des défauts optiques comprend dans cet exemple un ensemble d'éléments optiques 265, 267 permettant d'assurer la conjugaison du plan d'analyse 271 avec le plan 17 de la pupille d'entrée du système d'imagerie ainsi qu'un miroir de renvoi 266. Selon un exemple de réalisation, la lame 258 est une lame dichroïque et le faisceau d'analyse présente une longueur d'onde différente du ou des autres faisceaux réémis par la rétine pour l'imagerie, comme cela est décrit par la suite.

Un calculateur (non représenté) permet de déterminer les défauts optiques du système et d'envoyer une commande de correction au dispositif de correction 250, par exemple un miroir déformable de type Mirao 52-e Imagine Eyes®. Avantageusement, le calculateur, associé au Shack-Hartmann, détermine par rapport à des directions nominales la variation des directions des rayons lumineux après avoir traversé le système optique entaché de défauts optiques. Les variations ainsi mesurées peuvent être directement exploités pour le contrôle du miroir déformable. Le plan du miroir déformable est également conjugué optiquement avec le plan 17 de la pupille d'entrée du système d'imagerie.

D'autres dispositifs de correction du front d'onde peuvent être utilisés, comme par exemple un modulateur spatial de lumière à cristaux liquides (SLM pour « spatial light modulator ») ou un MEMs ou une lentille liquide multi-actionneurs.

Dans l'exemple des FIGS. 2A - 2C, le système d'imagerie à balayage multi-échelle comprend en outre un module d'éclairage adapté pour l'émission d'un faisceau d'éclairage de la rétine en vue de la mesure des défauts optiques.

Le module d'éclairage comprend dans cet exemple une source 261 d'éclairage de la rétine destinée à l'analyse des défauts optiques du système d'imagerie. La source 261 permet de former un point source secondaire sur la rétine de l'œil du sujet. Par exemple, la longueur d'onde centrale de la source d'éclairage 261 pour l'analyse des défauts optiques est de 750 nm, une telle longueur d'onde étant confortable pour le sujet et aussi proche que possible de la ou des longueur(s) d'onde d'imagerie. De préférence la longueur d'onde de la source 261 est différente de celle des sources lumineuses des modules d'éclairage et de détection 210, 220 pour des raisons de séparation des chemins optiques entre la mesure des défauts optiques et l'imagerie de la rétine. La source 261 est par exemple une diode laser ou plus avantageusement une diode super luminescente SLED. Une lame séparatrice 202 permet d'envoyer vers l'œil 10 du sujet les faisceaux lumineux d'éclairage de la rétine pour l'imagerie et pour l'analyse des défauts optiques. Un ensemble d'éléments optiques 262, 263, 264 permettent de former à partir de la source d'éclairage 261 un faisceau destiné à être focalisé sur la rétine. La lentille 263 peut être une lentille liquide à puissance variable (de type Varioptic® Artic® 25H0 par exemple) pour compenser l'amétropie de l'œil examiné.

Les FIGS 3A et 3B montrent des exemples de modules d'éclairage et de détection, adaptés à une imagerie de la rétine respectivement de type AOSLO et de type OCT.

La FIG. 3A représente un module d'éclairage et de détection 210 de type connu, adapté à une imagerie de la rétine respectivement de type AOSLO, et un module de balayage 231 pour le balayage d'un faisceau d'éclairage émis par le module 210 et d'un faisceau réémis par la rétine après illumination par ledit faisceau d'éclairage. Le module 210 comprend dans cet exemple une source lumineuse 211, par exemple une diode laser couplée à une fibre optique pour former un point source et une lentille optique 212 permettant de former à partir du point source un faisceau d'éclairage de diamètre $\Phi_{ecl}$ donné. Le module d'éclairage et de détection 210 comprend par ailleurs un trou de détection confocale 215 au travers duquel est focalisé grâce à une optique 214 le faisceau réémis par la rétine et renvoyé dans le module 210. Le module 210 comprend par ailleurs un détecteur 216, par exemple un photomultiplicateur ou une photodiode à avalanche, relié à une unité de traitement du signal 217, lui-même relié à un écran 218. Une lame partiellement réfléchissante 213 permet de séparer les faisceaux à l'émission et à la réception.

La FIG. 3B représente un module d'éclairage et de détection 220 de type connu, adapté à une imagerie de la rétine respectivement de type OCT, et un module de balayage 232 pour le balayage d'un faisceau d'éclairage émis par le module 220 et d'un faisceau réémis par la rétine après illumination par ledit faisceau d'éclairage. Le module 220 comprend dans cet exemple une source lumineuse 221 à faible cohérence temporelle, par exemple une source lumineuse de type SLED et un module de détection formé d'un interféromètre, par exemple un interféromètre fibré, par exemple de type Michelson, comprenant dans cet exemple un bras de référence fibré avec un élément de réflexion 225 et une optique 224. Un coupleur 223 reçoit les faisceaux issues des fibres 222-1, 222-2, 222-3 respectivement en provenance de la source, du bras de référence et de la rétine pour la formation d'interférences sur un détecteur 226, par exemple un photomultiplicateur ou une photodiode à avalanche ; Le module 220 comprend par ailleurs une unité de traitement du signal 227, elle-même reliée à un écran 228.

La FIG. 4 illustre un schéma le fonctionnement d'un autre exemple de réalisation d'un système d'imagerie 400 à balayage de la rétine selon la présente description.

Dans cet exemple, l'élément optique de renvoi permettant la séparation des voies petit-champ et grand-champ n'est pas une surface réfléchissante amovible mais une lame dichroïque 441. L'ensemble des éléments identiques à ceux décrits en référence aux FIGS. 2A - 2C sont indiqués avec des références identiques ne sont pas décrits à nouveau.

Pour faciliter la compréhension de la figure, on a supposé dans cet exemple que seul un module d'éclairage et de détection 410 est actif, par exemple un module d'éclairage et de détection adapté à une imagerie de la rétine respectivement de type AOSLO.

Dans cet exemple, le module d'éclairage et de détection 410 comprend un sous-module $410_A$ pour l'éclairage et la détection à une première longueur d'onde et sous-module $410_B$ pour l'éclairage et la détection à une deuxième longueur d'onde, différente de la première longueur d'onde.

Dans cet exemple, le faisceau d'éclairage à la première longueur d'onde, illustré en trait gras, et émis par le sous-module $410_A$, est balayé par le module de balayage 231 puis directement réfléchi sur la voie grand-champ comprenant le premier système optique 205, 201 au moyen de la lame dichroïque 441.

Le faisceau d'éclairage à la deuxième longueur d'onde, illustré en trait fin, et émis par le sous-module $410_B$, est balayé également par le module de balayage 231 puis transmis au moyen de la lame dichroïque 441 vers la voie petit-champ comprenant les deuxième (257, 256, 253) et troisième (205, 201) systèmes optiques.

Une lame partiellement réfléchissante 413 permet d'envoyer les faisceaux d'éclairage vers la rétine de l'œil et d'envoyer les faisceaux réémis par la rétine illuminée respectivement vers chacun des sous-modules.

Cette configuration permet d'avoir accès simultanément aux voies d'imagerie petit-champ et grand-champ avec le même module de balayage 231 et permet de ce fait une colocalisation très facile et très fiable des deux images. Le rapport entre la taille angulaire des deux champs est égal au rapport entre les tailles des diamètres des pupilles des deux faisceaux au niveau de la pupille de l'œil.

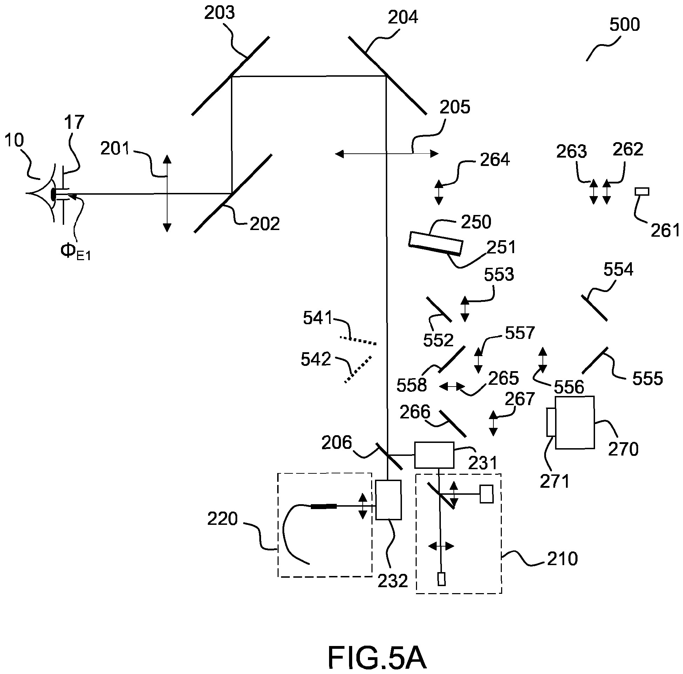

Les FIGS 5A - 5B illustrent le fonctionnement d'un autre exemple de réalisation d'un système d'imagerie à balayage de la rétine 500 selon la présente description.

Dans cet exemple, un premier élément optique de renvoi 541, au moins partiellement réfléchissant, permet d'envoyer un premier faisceau réémis par la rétine sur l'une et/ou l'autre des première et deuxième voies d'imagerie, et un deuxième élément optique de renvoi 542 permet d'envoyer le au moins un premier faisceau d'éclairage sur l'une et/ou l'autre des première et deuxième voies d'imagerie.

Là aussi, l'ensemble des éléments identiques à ceux décrits en référence aux FIGS. 2A - 2C sont indiqués avec des références identiques ne sont pas décrits à nouveau.

Par exemple, les premier et deuxième éléments optiques de renvoi 541, 542 sont des surfaces réfléchissantes amovibles solidaires. Alternativement, les premier et deuxième éléments optiques de renvoi 541, 542 peuvent être des lames dichroïques, comme décrit précédemment en référence à la FIG. 4.

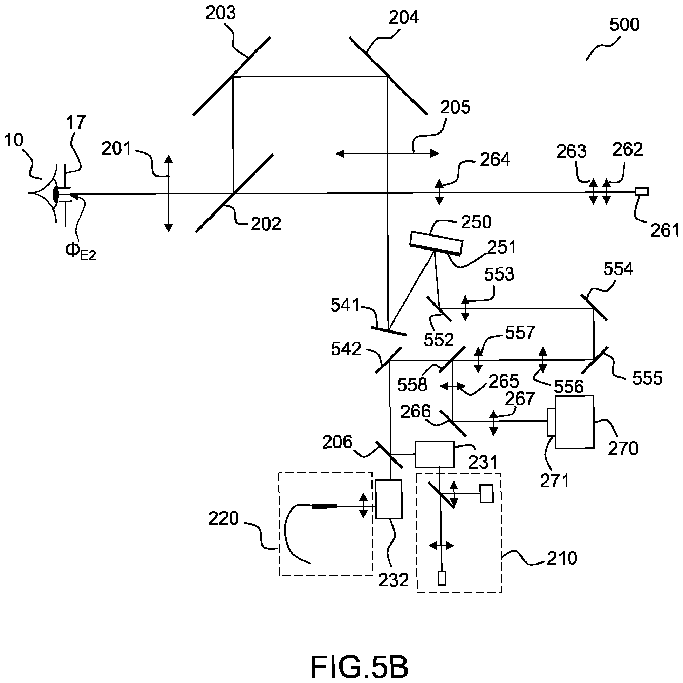

Ainsi, la FIG. 5A illustre le fonctionnement du système d'imagerie de la rétine lorsque la voie optique grand-champ est activée, avec le premier système optique 205, 201, ce qui est obtenu dans cet exemple lorsque les éléments de renvoi 541, 542 sont retirés. La FIG. 5B illustre le fonctionnement du système d'imagerie de la rétine lorsque la voie optique petit-champ est activée, ce qui est obtenu dans cet exemple lorsque les éléments de renvoi 541, 542 sont mis en place. Cette configuration particulière permet notamment de limiter le nombre d'éléments réfléchissants dans la voie grand-champ.

La voie « petit champ » comprend, comme précédemment, un dispositif de correction de front d'onde 250 présentant une surface utile 251 de dimension donnée, et un deuxième système optique, destiné à conjuguer un plan de rotation du module de balayage et la surface utile 251 et présentant un deuxième grandissement $g_2$. Le deuxième système optique comprend dans l'exemple des FIGS. 5A - 5B un ensemble d'éléments optiques 557, 556, 553. La voie « petit champ » comprend en outre, comme précédemment, un troisième système optique adapté pour conjuguer la surface utile 251 du dispositif de correction et le plan 17 de la pupille d'entrée de l'œil 10, présentant un troisième grandissement $g_3$. Le troisième système optique comprend dans cet exemple les éléments optiques 205, 201 formant le premier système optique. La voie petit-champ comprend par ailleurs, un ensemble d'éléments optiques de renvoi, dans cet exemple 555, 554, 552, pour diriger le faisceau d'éclairage vers l'œil 10 d'un patient. Une lame partiellement-réfléchissante 558, par exemple une lame dichroïque, permet comme précédemment de défléchir le faisceau réémis par la rétine à la longueur d'onde de la source d'analyse 261 vers le module d'analyse des défauts optiques.

Bien que décrite à travers un certain nombre d'exemples de réalisation détaillés, le dispositif d'imagerie rétinienne et la méthode selon l'invention comprennent différentes variantes, modifications et perfectionnements qui apparaîtront de façon évidente à l'homme de l'art, étant entendu que ces différentes variantes, modifications et perfectionnements font partie de la portée de l'invention, telle que définie par les revendications qui suivent.

Notamment, un seul module d'éclairage et de détection peut être utilisé, auquel cas la lame partiellement réfléchissante 206 illustré sur les FIGS 2A - 2C, 4, 5A - 5B n'est plus nécessaire. Par ailleurs, des exemples de systèmes d'imagerie à balayage de la rétine ont été décrits avec un module d'éclairage et de détection adapté à une imagerie de la rétine de type AOSLO ou OCT. La présente description peut s'appliquer généralement à tout dispositif d'imagerie par balayage de la rétine avec optique adaptative.

Le module d'éclairage de la rétine en vue de l'analyse des défauts optiques est optionnel, l'analyse des défauts pouvant être réalisée sur un faisceau réémis par la rétine, résultant de l'éclairage par un des modules d'éclairage et de détection 210, 220. Il est également possible de s'affranchir d'un module d'analyse du front d'onde, par exemple en mettant en œuvre un procédé de correction qui se base sur un critère de qualité des images acquises, bien que l'utilisation d'un module d'analyse du front d'onde soit préférable pour des raisons de vitesse d'asservissement.

Par ailleurs, les systèmes optiques ont été représentés à des fins illustratives et peuvent être modifiés en fonction des besoins spécifiques de l'application ; par exemple, les systèmes optiques réfractifs peuvent être remplacés par des systèmes optiques réflectifs.

Enfin, dans l'exemple des FIGS 2A - 2C, la voie d'imagerie « grand-champ » est activée lorsque le ou les élément(s) de renvoi sont mis en place et la voie d'imagerie « petit-champ » est activée lorsque le ou les élément(s) de renvoi sont retirés mais le système optique pourrait facilement être conçu pour que ce soit l'inverse (comme dans le cas des FIGS 5A-5B).

**Revendications**

1. Système (200) d'imagerie à balayage de la rétine multi-échelle comprenant :

   - au moins un premier module d'éclairage et de détection (210) configuré pour l'émission d'au moins un premier faisceau d'éclairage avec un diamètre de dimension donnée ($\Phi_{ecl}$) et la détection d'au moins un premier faisceau réémis par la rétine;
   - au moins un premier module de balayage (231) du au moins un premier faisceau d'éclairage et du au moins un premier faisceau réémis;
   - une première voie optique, dite voie « grand champ », pour la focalisation du au moins un premier faisceau d'éclairage sur la rétine et la réception du au moins un premier faisceau réémis par la rétine, comprenant :
      ◦ un premier système optique (205, 201) avec un premier grandissement ($g_1$), configuré pour

conjuguer un plan situé à proximité d'un plan de rotation du au moins un module de balayage et le plan (17) de la pupille d'entrée de l'œil (10);
   - une deuxième voie optique, dite voie « petit champ », pour la focalisation du au moins un premier faisceau d'éclairage sur la rétine et la réception du au moins un premier faisceau réémis par la rétine, comprenant :

      ◦ un dispositif (250) de correction de front d'onde présentant une surface utile (251) de dimension donnée ($\Phi_{DM}$);
      ◦ un deuxième système optique (257, 256, 253) avec un deuxième grandissement ($g_2$) configuré pour conjuguer un plan situé à proximité d'un plan de rotation du au moins un premier module de balayage et la surface utile du dispositif de correction de front d'onde,
      ◦ un troisième système optique avec un troisième grandissement ($g_3$), comprenant au moins une partie du premier système optique, configuré pour conjuguer ladite surface utile (251) du dispositif de correction et le plan (17) de la pupille d'entrée de l'œil (10);

   - au moins un premier élément optique de renvoi (241, 242), au moins partiellement réfléchissant, configuré pour envoyer le au moins un premier faisceau réémis par la rétine sur l'une et/ou l'autre des première et deuxième voies d'imagerie, et destiné à être positionné sur la première voie d'imagerie, entre la partie commune (201, 205) des premier et troisième systèmes optiques et le au moins un premier module de balayage (210), et sur la deuxième voie d'imagerie, entre la partie commune des premier et troisième systèmes optiques et le dispositif de correction de front d'onde.

2. Système d'imagerie à balayage multi-échelle selon la revendication 1, dans lequel ledit premier élément optique de renvoi est configuré en outre pour envoyer le au moins un premier faisceau d'éclairage sur l'une et/ou l'autre des première et deuxième voies d'imagerie.

3. Système d'imagerie à balayage multi-échelle selon la revendication 1, dans lequel un deuxième élément optique de renvoi est configuré pour envoyer le au moins un premier faisceau d'éclairage sur l'une et/ou l'autre des première et deuxième voies d'imagerie.

4. Système d'imagerie à balayage multi-échelle selon l'une des revendications précédentes, dans lequel ledit au moins un premier élément optique de renvoi est une surface réfléchissante amovible, configurée pour basculer entre l'une des première et deuxième voies d'imagerie et l'autre.

5. Système d'imagerie à balayage multi-échelle selon l'une des revendications précédentes, dans lequel ledit au moins un premier élément optique de renvoi est une lame dichroïque, configurée pour envoyer le au moins un premier faisceau réémis par la rétine sur l'une desdites première et deuxième voies d'imagerie et envoyer au moins un deuxième faisceau réémis par la rétine, de longueur d'onde différente de celle dudit au moins un premier faisceau réémis par la rétine, sur l'autre voie d'imagerie.

6. Système d'imagerie à balayage multi-échelle selon l'une des revendications précédentes, dans lequel ledit au moins un premier module d'éclairage et de détection comprend au moins une source lumineuse ponctuelle (211) et au moins un système de détection confocale et est configuré pour une imagerie de la rétine de type AOSLO.

7. Système d'imagerie à balayage multi-échelle selon l'une des revendications précédentes, dans lequel ledit au moins un premier module d'éclairage et de détection comprend au moins une source lumineuse ponctuelle (221) et au moins un interféromètre et est configuré pour une imagerie de la rétine de type OCT.

8. Système d'imagerie à balayage multi-échelle selon l'une des revendications précédentes, comprenant en outre :

   - un deuxième module d'éclairage et de détection (220) configuré pour l'émission d'au moins un deuxième faisceau d'éclairage avec un diamètre de dimension donnée et la détection d'au moins un deuxième faisceau réémis par la rétine;
   - un deuxième module de balayage (232) du au moins un deuxième faisceau d'éclairage et du au moins un deuxième faisceau réémis.

9. Système d'imagerie à balayage multi-échelle selon l'une des revendications précédentes, comprenant en outre un module d'analyse du front d'onde, configuré pour l'analyse d'au moins une partie des défauts optiques d'un faisceau réémis par la rétine et envoyé sur la deuxième voie d'imagerie.

10. Système d'imagerie à balayage multi-échelle selon la revendication 9, dans lequel le module d'analyse du front d'onde comprend un analyseur de type Shack-Hartmann.

11. Système d'imagerie à balayage multi-échelle selon l'une quelconque des revendications 9 ou 10, comprenant en outre un module d'éclairage configuré pour l'émission d'un faisceau d'éclairage de la rétine en vue de la mesure des défauts optiques.

12. Méthode d'imagerie par balayage de la rétine au moyen d'un système d'imagerie selon l'une quelconque des revendications précédentes.

13. Méthode d'imagerie par balayage de la rétine selon la revendication 12, dans laquelle

   - ledit au moins un premier élément optique de renvoi est une surface réfléchissante amovible ; et la méthode comprend :

      - le retrait dudit au moins un premier élément optique de renvoi pour basculer de l'une desdites première et deuxième voies d'imagerie à l'autre voie d'imagerie.

14. Méthode d'imagerie par balayage de la rétine selon la revendication 12, dans laquelle

   - ledit au moins un premier élément optique de renvoi est une lame dichroïque ; la méthode comprenant :

      - l'envoi du au moins un premier faisceau réémis par la rétine sur l'une desdites première et deuxième voies d'imagerie et l'envoi d'au moins un deuxième faisceau réémis par la rétine sur l'autre voie d'imagerie, lesdits premier et deuxième faisceaux réémis par la rétine ayant des longueurs d'onde différentes.

**Patentansprüche**

1. System (200) zur mehrskaligen Netzhautrasterbildgebung, aufweisend:

   - mindestens ein erstes Leucht- und Detektionsmodul (210), das so konfiguriert ist, dass es mindestens einen ersten Lichtstrahl mit einem Durchmesser gegebener Größe ($\Phi_{ecl}$) aussendet und mindestens einen ersten von der Netzhaut zurückausgestrahlten Lichtstrahl detektiert;
   - mindestens ein erstes Abtastmodul (231) zum Abtasten des mindestens einen ersten Lichtstrahls und des mindestens einen ersten zurückausgestrahlten Lichtstrahls;
   - einen ersten optischen Weg, den sogenannten "Großfeld"-Weg, zum Fokussieren des mindestens einen ersten Lichtstrahls auf die Netzhaut und zum Empfangen des mindestens einen ersten von der Netzhaut zurückausgestrahlten Lichtstrahls, aufweisend:

- ein erstes optisches System (205, 201) mit einer ersten Vergrößerung ($g_1$), die so konfiguriert ist, dass sie eine Ebene, die sich in der Nähe einer Rotationsebene des mindestens einen Abtastmoduls befindet, und die Ebene (17) der Eintrittspupille des Auges (10) konjugiert;

- einen zweiten optischen Weg, den sogenannten "Kleinfeld"-Weg, zum Fokussieren des mindestens einen ersten Lichtstrahls auf die Netzhaut und zum Empfangen des mindestens einen ersten von der Netzhaut zurückausgestrahlten Lichtstrahls, aufweisend:

- eine Vorrichtung (250) zur Korrektur der Wellenfront, die eine Nutzfläche (251) mit einer gegebenen Größe ($\Phi_{DM}$) aufweist;
- ein zweites optisches System (257, 256, 253) mit einer zweiten Vergrößerung ($g_2$), die so konfiguriert ist, dass sie eine Ebene in der Nähe einer Rotationsebene des mindestens einen ersten Abtastmoduls und die Nutzfläche der Wellenfrontkorrekturvorrichtung konjugiert,
- ein drittes optisches System mit einer dritten Vergrößerung ($g_3$), das mindestens einen Teil des ersten optischen Systems aufweist und so konfiguriert ist, dass es die Nutzfläche (251) der Korrekturvorrichtung und die Ebene (17) der Eintrittspupille des Auges (10) konjugiert;
- mindestens ein erstes, mindestens teilweise reflektierendes optisches Umlenkelement (241, 242), das so konfiguriert ist, dass es den mindestens einen ersten, von der Netzhaut zurückgeworfenen Strahl auf den einen und/oder den anderen der ersten und zweiten Bildgebungspfade richtet, und das dazu bestimmt ist, auf dem ersten Bildgebungspfade zwischen dem gemeinsamen Teil (201, 205) des ersten und dritten optischen Systems und dem mindestens einen ersten Abtastmodul (210) und auf dem zweiten Bildgebungspfade zwischen dem gemeinsamen Teil des ersten und dritten optischen Systems und der Wellenfrontkorrekturvorrichtung positioniert zu werden.

2. Mehrskalen- Rasterbildgebungssystem nach Anspruch 1, wobei das erste optische Umlenkelement ferner so konfiguriert ist, dass es den mindestens einen ersten Lichtstrahl auf den einen und/oder den anderen der ersten und zweiten Bildgebungspfade richtet.

3. Mehrskalen-Rasterbildgebungssystem nach Anspruch 1, wobei ein zweites optisches Umlenkelement so konfiguriert ist, dass es den mindestens einen ersten Lichtstrahl auf den einen und/oder den anderen der ersten und zweiten Bildgebungspfade richtet.

4. Mehrskalen-Rasterbildgebungssystem nach einem der vorhergehenden Ansprüche, wobei das mindestens eine erste optische Umlenkelement eine entfernbare reflektierende Oberfläche ist, die so konfiguriert ist, dass sie zwischen dem ersten und zweiten Bildgebungskanal und dem anderen umschaltet.

5. Mehrskalen-Rasterbildgebungssystem nach einem der vorhergehenden Ansprüche, wobei das mindestens eine erste optische Umlenkelement eine dichroitische Lamelle ist, die so konfiguriert ist, dass sie den mindestens einen ersten von der Netzhaut zurückausgestrahlten Lichtstrahl auf einen der ersten und zweiten Bildgebungspfade richtet und mindestens einen zweiten von der Netzhaut zurückausgestrahlten Lichtstrahl mit einer Wellenlänge, die sich von der des mindestens einen ersten von der Netzhaut zurückausgestrahlten Lichtstrahls unterscheidet, auf den anderen Bildgebungspfad richtet.

6. Mehrskalen-Rasterbildgebungssystem nach einem der vorhergehenden Ansprüche, wobei das mindestens eine erste Leucht- und Detektionsmodul mindestens eine Punktlichtquelle (211) und mindestens ein konfokales Detektionssystem aufweist und für eine Bildgebung der Netzhaut durch adaptive Rasterlaserophthalmoskopie, auch AOSLO genannt, konfiguriert ist.

7. Mehrskalen-Rasterbildgebungssystem nach einem der vorhergehenden Ansprüche, wobei das mindestens eine erste Leucht- und Detektionsmodul mindestens eine Punktlichtquelle (221) und mindestens ein Interferometer aufweist und für eine Bildgebung der Netzhaut durch optische Kohärenztomographie, auch OCT genannt, konfiguriert ist.

8. Mehrskalen-Rasterbildgebungssystem nach einem der vorhergehenden Ansprüche, ferner aufweisend:

- ein zweites Leucht- und Detektionsmodul (220), das so konfiguriert ist, dass es mindestens einen zweiten Lichtstrahl mit einem Durchmesser gegebener Größe aussendet und mindestens einen zweiten Strahl detektiert, der von der Netzhaut zurückausgestrahlt wird;
- ein zweites Modul (232) zum Abtasten des mindestens einen zweiten Lichtstrahls und des mindestens einen zweiten zurückausgestrahlten Lichtstrahls.

**9.** Mehrskalen-Rasterbildgebungssystem nach einem der vorhergehenden Ansprüche, ferner mit einem Wellenfront-Analysemodul, das zur Analyse mindestens eines Teils der optischen Defekte eines von der Netzhaut zurückausgestrahlten und auf den zweiten Bildgebungspfad gerichteten Lichtstrahl konfiguriert ist.

**10.** Mehrskalen-Rasterbildgebungssystem nach Anspruch 9, wobei das Wellenfront-Analysemodul einen Analysator vom Shack-Hartmann-Typ aufweist.

**11.** Mehrskalen-Rasterbildgebungssystem nach einem der Ansprüche 9 oder 10, das außerdem ein Leuchtmodul aufweist, das so konfiguriert ist, dass es einen Lichtstrahl zur Messung optischer Defekte auf die Netzhaut aussendet.

**12.** Verfahren zur Bildgebung durch Abtasten der Netzhaut mithilfe eines Bildgebungssystems nach einem der vorhergehenden Ansprüche.

**13.** Verfahren zur Bildgebung durch Abtasten der Netzhaut nach Anspruch 12, wobei

- das mindestens eine erste optische Umlenkelement eine entfernbare reflektierende Oberfläche ist; und das Verfahren aufweist:

- Entfernen des mindestens einen ersten optischen Umlenkelements, um von einem der ersten und zweiten Bildgebungspfade auf den anderen Bildgebungspfad umzuschalten.

**14.** Verfahren zur Bildgebung durch Abtasten der Netzhaut nach Anspruch 12, wobei

- das mindestens eine erste optische Umlenkelement eine dichroitische Lamelle ist; wobei das Verfahren aufweist:

- richten des mindestens einen ersten von der Netzhaut zurückausgestrahlten Lichtstrahls auf einen der ersten und zweiten Bildgebungspfade und richten des mindestens einen zweiten von der Netzhaut zurückausgestrahlten Lichtstrahls auf den anderen Bildgebungspfad, wobei die ersten und zweiten von der Netzhaut zurückausgestrahlten Lichtstrahlen unterschiedliche Wellenlängen aufweisen.

**Claims**

**1.** A multi-scale scanning imaging system (200) of the retina comprising:

- at least one first lighting and detection module (210) configured for emitting at least one first lighting beam with a diameter of given dimension ($\Phi_{ecl}$) and for detecting at least one first beam reemitted by the retina;
- at least one first scanning module (231) of the at least one first lighting beam and the at least one first reemitted beam;
- a first optical path, referred to as a "wide field" path, for focusing the at least one first lighting beam on the retina and for receiving the at least one first beam reemitted by the retina, comprising:

o a first optical system (205, 201) with a first magnification ($g_1$) configured to conjugate a plane located near a plane of rotation of the at least one scanning module and the plane (17) of the entrance pupil of the eye (10);

- a second optical path, referred to as a "small field" path, for focusing the at least one first lighting beam on the retina and for receiving the at least one first beam reemitted by the retina, comprising:

o a wavefront correction device (250) having an effective surface (251) of given dimension ($\Phi_{DM}$);
o a second optical system (257, 256, 253) with a second magnification ($g_2$) configured to conjugate a plane located near a plane of rotation of the at least one first scanning module and the effective surface of the wavefront correction device,
o a third optical system with a third magnification ($g_3$), comprising at least a part of the first optical system, configured to conjugate said effective surface (251) of the correction device and the plane (17) of the entrance pupil of the eye (10);

- at least one first optical deflection element (241, 242), at least partially reflective, configured to send the at least one first beam reemitted by the retina on one and/or the other of the first and second imaging paths and intended to be positioned on the first imaging path, between the common part (201, 205) of the first and third optical systems and the at least one first scanning module (210), and on the second imaging path, between the common part of the first and third optical systems and the wavefront correction device.

**2.** The multi-scale scanning imaging system according to claim 1, wherein said first optical deflection element is further configured to send the at least one first lighting beam on one and/or the other of the first and second imaging paths.

**3.** The multi-scale scanning imaging system according to claim 1, wherein a second optical deflection element is configured to send the at least one first lighting beam on one and/or the other of the first and second imaging paths.

**4.** The multi-scale scanning imaging system according to one of the preceding claims, wherein said at least one first optical deflection element is a removable reflecting surface configured to switch between one of the first and second imaging paths and the other.

**5.** The multi-scale scanning imaging system according to one of the preceding claims, wherein said at least one first optical deflection element is a dichroic plate, configured to send the at least one first beam reemitted by the retina on one of said first and second imaging paths and to send at least one second beam reemitted by the retina, of a wavelength that it different from that of said at least one first beam reemitted by the retina, on the other imaging path.

**6.** The multi-scale scanning imaging system according to one of the preceding claims, wherein said at least one first lighting and detection module comprises at least one point light source (211) and at least one confocal detection system and is configured for AOSLO type imaging of the retina.

**7.** The multi-scale scanning imaging system according to one of the preceding claims, wherein said at least one first lighting and detection module comprises at least one point light source (221) and at least one interferometer and is configured for OCT type imaging of the retina.

**8.** The multi-scale scanning imaging system according to one of the preceding claims, further comprising:

- a second lighting and detection module (220) configured for emitting at least one second lighting beam with a diameter of given dimension and for detecting at least one second beam reemitted by the retina;
- a second scanning module (232) of the at least one second lighting beam and the at least one second reemitted beam.

**9.** The multi-scale scanning imaging system according to one of the preceding claims, further comprising a wavefront analysis module configured to analyse at least part of the optical defects of a beam reemitted by the retina and sent on the second imaging path.

**10.** The multi-scale scanning imaging system according to claim 9, wherein the wavefront analysis module comprises a Shack-Hartmann type analyser.

**11.** The multi-scale scanning imaging system according to any one of claims 9 or 10, further comprising a lighting module configured for emitting a lighting beam for lighting the retina for the measurement of the optical defects.

**12.** A scanning imaging method of the retina using an imaging system according to any one of the preceding claims.

**13.** The scanning imaging method of the retina according to claim 12, wherein

- said at least one first optical deflection element is a removable reflecting surface; and the method comprises:

- removing said at least one first optical deflection element to switch from one of said first and second imaging paths to the other imaging path.

**14.** The scanning imaging method of the retina according to claim 12, wherein

- said at least one first optical deflection element is a dichroic plate; the method comprising:

- sending the at least one first beam reemitted by the retina on one of said first and second imaging paths and sending at least one second beam reemitted by the retina on the other imaging path, said first and second beams reemitted by the retina having different wavelengths.

10  17  16  13  14  12

OPTIQUE

SCANNING

CORRECTION

DETECTION

MESURE DES
DEFAUTS
OPTIQUES

15

ECLAIRAGE

11

## FIG.1A

10  17  16  13  14  12

OPTIQUE

SCANNING

CORRECTION

DETECTION
OCT

MESURE DES
DEFAUTS
OPTIQUES

15

ECLAIRAGE

11

## FIG.1B

# FIG.2A

FIG.2B

FIG.2C

FIG.3A

FIG.3B

FIG.4

FIG.5A

FIG.5B

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2016009603 A
- US 7758189 B
- WO 2014053824 A

**Littérature non-brevet citée dans la description**

- **A. ROORDA et al.** Adaptive optics scanning laser ophtalmoscopy. *Optics express 405,* 2002, vol. 10 (9
- **R. ZAWADZKI.** Adaptive-optics optical coherence tomography for high resolution and high speed 3D retinal in vivo imaging. *Optics Express 8532,* 2005, vol. 13 (21
- **D'A. DUBRA et al.** First-order design of a reflective viewfinder for adaptive optics ophtalmoscopy. *Optics Express,* 2012, vol. 20 (24